# EUROPEAN PATENT APPLICATION

(11) **EP 2 870 963 A1**
(43) Date of publication of application: **13.05.2015**
(21) Application number: 13813145.3
(22) Date of filing: 08.07.2013
(51) Int. Cl.: A61K 9/70, A61K 31/381

(54) **PREPARATION FOR PERCUTANEOUS ABSORPTION CONTAINING ROTIGOTINE**

(30) Priority: 06.07.2012 KR 20120073798
(71) Applicant: SK Chemicals Co., Ltd., Seongnam-si, Gyeonggi-do 463-400 (KR)
(72) Inventor: KIM, Hye-Min, Anyang-si Gyeonggi-do 430-010 (KR); HWANG, Yong-Youn, Suwon-si Gyeonggi-do 440-210 (KR); YOUN, Won-No, Seoul 151-015 (KR); PARK, Yeo-Jin, Seoul 131-200 (KR); OH, Joon-Gyo, Suwon-si Gyeonggi-do 440-709 (KR); IM, Jong-Seob, Suwon-si Gyeonggi-do 440-824 (KR); KIM, Hun-Taek, Seoul 137-951 (KR)
(74) Representative: Gulde & Partner
(86) International application number: PCT/KR2013/006060
(87) International publication number: WO 2014/007597

(57) **Abstract**

The present invention relates to a method for preparing a preparation for percutaneous absorption, which comprises rotigotine as an active ingredient, and more specifically, to a method for preparing a preparation for percutaneous absorption comprising a step of mixing rotigotine and an ethylene-vinyl acetate adhesive so as to have a weight ratio of 1:0.1 to 20, a preparation for percutaneous absorption manufactured by the method, and a percutaneous treatment system. The preparation and the system, according to the present invention, prevent separation of the rotigotine, thereby increasing long-term storage stability, and effectively release the rotigotine, and thus can be effectively applied to preparing patch medication containing the rotigotine.

## Description

### Technical Field

This application claims priority from and the benefit of Korean Patent Application No. 10-2012-0073798 filed on July 6, 2012, which is hereby incorporated by reference for all purposes as if fully set forth herein.

The present invention relates to a method for preparing a transdermally absorbable preparation comprising rotigotine as an active ingredient, and more particularly, to a method for preparing a transdermally absorbable preparation, the method including a step of mixing rotigotine and an ethylene-vinyl acetate adhesive at a weight ratio of 1 : 0.1 to 20, to a transdermally absorbable preparation prepared by the method, and to a transdermally therapeutic system.

### Background Art

Rotigotine, (-)-5,6,7,8-tetrahydro-6-[propyl-[2-(2-thienyl)ethyl]amino]-1-naphtahlenol, is used for the treatment of Parkinson's disease(PD) and restless legs syndrome (RLS), as a non-ergoline dopamine agonist.

This rotigotine has been commercially available in a patch dosage form. A patch-type transdermal composition is disclosed in U.S. Patent No. US 7413747 B. Also, one of the commercially available patches employs a silicone adhesive, which is disclosed in EP 1033978B. However, the commercially available patches cause the precipitation of rotigotine crystals in room-temperature storage conditions, and thus it is difficult to secure the storage period necessary for commercial distribution of the commercially available patches. In order to solve the problem, patent WO 2011/076879 discloses a rotigotine transdermal composition with an added polymer, such as polyvinylpyrrolidone.

Rotigotine transdermal composition products on the market contain polyvinylpyrrolidone, but polyvinylpyrrolidone serves to increase the viscosity of a drug storage layer. As a result, the coating may not be uniform, and the uniform mixing of the drug storage layer is difficult. Thus, measures capable of preventing crystallization to solve the problems are being constantly researched.

### Detailed Description of the Invention

### Technical Problem

The present inventors, while researching methods for improving the storage stability of rotigotine patches, have found that rotigotine crystals are not formed when a patch is prepared by mixing rotigotine with ethylene-vinyl acetate adhesive, and thus have completed the present invention.

Therefore, an aspect of the present invention is to provide a method for preparing a transdermally absorbable preparation, the method including a step of mixing rotigotine and an ethylene-vinyl acetate adhesive at a weight ratio of 1 : 0.1 to 20.

Another aspect of the present invention is to provide a transdermally absorbable preparation prepared by the method.

Still another aspect of the present invention is to provide a transdermally therapeutic system including: a drug-containing matrix layer containing rotigotine and an ethylene-vinyl acetate adhesive at a weight ratio of 1 : 0.1 to 20; and a substrate for supporting the drug-containing matrix layer.

### Technical Solution

In accordance with an aspect of the present invention, there is provided a method for preparing a transdermally absorbable preparation, the method including a step of mixing rotigotine and an ethylene-vinyl acetate (EVA) adhesive at a weight ratio of 1 : 0.1 to 20.

In accordance with another aspect of the present invention, there is provided a transdermally absorbable preparation prepared by the method.

In accordance with still another aspect of the present invention, there is provided a transdermal therapeutic system including: a drug-containing matrix layer containing rotigotine and an ethylene-vinyl acetate adhesive at a weight ratio of 1 : 0.1 to 20; and a substrate for supporting the drug-containing matrix layer.

Hereinafter, the present invention will be described.

The present invention provides a method for preparing a transdermally absorbable preparation, the method including a step of mixing rotigotine and an ethylene-vinyl acetate (EVA) adhesive at a weight ratio of 1 : 0.1 to 20.

Rotigotine ((-)-5,6,7,8-tetrahydro-6-[propyl-[2-(2-thienyl)ethyl]amino]-1-naphtahlenol) has a structure of chemical formula 1 below, and is used for the treatment of Parkinson's disease(PD) and restless legs syndrome(RLS), as a non-ergoline dopamine agonist. Rotigotine of the present invention includes a compound represented by chemical formula 1 below and a salt.

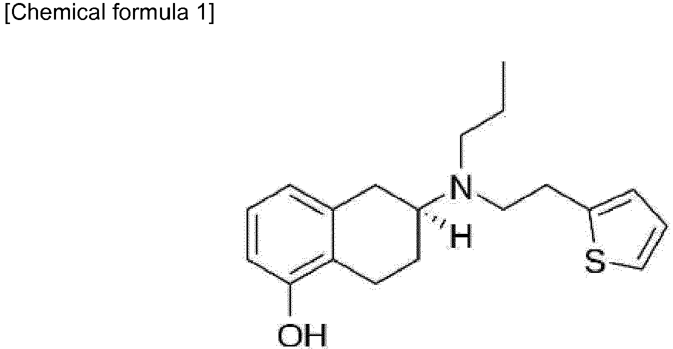

Ethylene-vinyl acetate (EVA) is a copolymer of ethylene and vinyl acetate, and physical properties thereof vary depending on the degree of polymerization and the content of vinyl acetate. EVA having a less vinyl acetate content is generally processed like polyethylene (PE), and has favorable printability and thus is used for a film or laminate, a foaming product, a bag for heavy weight packaging, and the like. EVA having 10-20% of vinyl acetate is blended with PVC or other resins to improve moldability and flexibility at low temperatures. A copolymer having a large content of vinyl acetate is an adhesive having excellent aging stability or weather resistance, and is a main material source of, particularly, a hot-melt type adhesive.

The vinyl acetate unit of the ethylene-vinyl acetate copolymer used herein is particularly not limited, and may contain, for example, 35% to 80% of vinyl acetate units, and preferably 40% to 80% of vinyl acetate units.

The preparing method of the present invention is characterized in that rotigotine and the ethylene-vinyl-acetate adhesive are mixed at a weight ratio of 1 : 0.1 to 20, and the weight percentage of the ethylene-vinyl-acetate adhesive per 1 part by weight of rotigotine may be selected from, for example, 0.1 to 1, 0.1 to 2, 0.1 to 3, 0.1 to 4, 0.1 to 5, 0.1 to 6, 0.1 to 7, 0.1 to 8, 0.1 to 9, 0.1 to 10, 0.1 to 11, 0.1 to 12, 0.1 to 13, 0.1 to 14, 0.1 to 15, 0.1 to 16, 0.1 to 17, 0.1 to 18, 0.1 to 19, 0.1 to 20, 0.2 to 10, 0.3 to 10, 0.4 to 10, 0.5 to 10, 0.6 to 10, 0.7 to 10, 0.8 to 10, 0.9 to 10, 1.0 to 10, 1.1 to 10, 1.2 to 10, 1.3 to 10, 1.4 to 10, 1.5 to 10, 1.6 to 10, 1.7 to 10, 1.8 to 10, 1.9 to 10, 2 to 10, 3 to 10, 4 to 10, 5 to 10, 6 to 10, 7 to 10, 8 to 10, 9 to 10, 0.2 to 20, 0.3 to 20, 0.4 to 20, 0.5 to 20, 0.6 to 20, 0.7 to 20, 0.8 to 20, 0.9 to 20, 1.0 to 20, 1.1 to 20, 1.2 to 20, 1.3 to 20, 1.4 to 20, 1.5 to 20, 1.6 to 20, 1.7 to 20, 1.8 to 20, 1.9 to 20, 2 to 20, 3 to 20, 4 to 20, 5 to 20, 6 to 20, 7 to 20, 8 to 20, 9 to 20, 10 to 20, and 15 to 20. Of these weight percentages, 0.3 to 20 may be preferable.

When the weight percentage of EVA is lower than the minimum value, the stability of rotigotine deteriorates, causing the precipitation of crystals during the long-term storage, and the adhesive strength deteriorates and thus the adhesive is hardly attached to the skin. When the weight percentage of EVA is higher than the maximum value, the skin permeability deteriorates, and the skin adherence is too strong, causing the adhesive to be an irritant to the skin when it is attached to and then detached from the skin. According to the preparing method of the present invention in which rotigotine and ethylene-vinyl acetate are mixed at a ratio of 1 : 0.1 to 20, a transdermally absorbable preparation, which causes no rotigotine crystal and has excellent skin permeation and significantly improved long-term stability and permeability, can be prepared.

This fact is first presented by the present inventors.

In an example of the present invention, the stability of rotigotine was tested by mixing various adhesive materials and rotigotine and allowing the mixtures to stand for one day. The results confirmed that the precipitation or layer separation did not occur in cases where ethylene-vinyl acetate was used together with some adhesives.

In another example of the present invention, transdermal administration systems (patches) were manufactured using adhesives causing no precipitation or layer separation at the time of mixing with rotigotine in the above example, and then long-term stability of the transdermal administration systems was evaluated. A matrix layer is formed by mixing each of the adhesives and rotigotine, and then the matrix layer is placed under accelerated test conditions. The observation results confirmed that, in cases where the ethylene-vinyl acetate adhesive of the present invention is used or an acryl adhesive is used, the crystals were not precipitated even within four weeks.

In still another example of the present invention, the skin permeation of the transdermal administration systems using various adhesives was measured. The results confirmed that the transdermal administration system using the acryl adhesive had low skin permeation and thus was not suitable, and the transdermal administration system using the ethylene-vinyl acetate adhesive showed a permeation ratio equal to that of the silicone adhesive used as an adhesive of an existing rotigotine patch.

According to the preparing method of the present invention, it was confirmed that a transdermally absorbable preparation with rotigotine, which has excellent skin permeation and long-term storage ability, was prepared.

Thus, the present invention provides a transdermally absorbable preparation prepared by the method of the present invention.

The transdermally absorbable preparation of the present invention is characterized by being prepared by a method including a step of mixing rotigotine and an ethylene-vinyl acetate adhesive at a weight ratio of 1 : 0.1 to 20. In the transdermally absorbable preparation of the present invention, the ratio of rotigotine and the ethylene-vinyl acetate adhesive is as described in the method for preparing a transdermally absorbable preparation.

The transdermally absorbable preparation of the present invention comprises rotigotine as an active drug ingredient, can conveniently administer the drug into the human body in a skin patch manner, and can constantly maintain a drug effect for a long time. In addition, in cases where the ethylene-vinyl acetate adhesive is mixed with rotigotine at a particular ratio, the rotigotine is not crystallized, thereby stably storing the transdermally absorbable preparation for a long time. The rotigotine and ethylene-vinyl acetate adhesive, which are the active ingredients of the transdermally absorbable preparation of the present invention, are as described as above.

In addition, the transdermally absorbable preparation of the present invention may further contain a skin permeation promoter, a vehicle, an abirritant, an adhesive, a stabilizer, or a carrier.

The skin permeation promoter is for promoting the permeation of a drug into the skin, and may include, for example, hydrophilic organic solvent, aprotic solvent, fatty acid, fatty alcohol, fatty acid ester, pyrrolidone, essential oil, surfactant, phospholipids, and the like.

When the adhesive strength is not enough using only EVA, another adhesive may be further added. The adhesive may be an acryl based adhesive, a rubber based adhesive, an ethylene-vinyl acetate adhesive, a styrene-based adhesive, a silicone based adhesive, or the like, and may be preferably a styrene based adhesive.

The stabilizer is a treatment agent for preventing the drug separation, and a normally used pharmaceutically acceptable alkalifying agent and anti-oxidant may be used as the stabilizer contained in the transdermally absorbable preparation of the present invention. An acceptable stabilizer may include, but is not limited to, butylated hydroxytoluene (BHT), dibutyl hydroxy toluene (DHT), butylated hydroxyanisole (BHA), sodium sulfite, sodium pyrosulfite, sodium hydrogen sulfite, benzoic acid sodium, sodium metabisulfite, propyl gallate, calcium phosphate, tocopherol and its ester, and the like.

The carrier contained in the transdermally absorbable preparation of the present invention is a biocompatible carrier, and may be, but is not limited to, a carrier for parenteral administration. The carrier for parenteral administration may include water, appropriate oil, a saline solution, aqueous glucose, glycol, and the like. For other pharmaceutically acceptable carriers, one disclosed in the following document may be referenced (Remington's Pharmaceutical Sciences, 19th ed., Mack Publishing Company, Easton, PA, 1995). The concentration of the biocompatible carrier may be, but is not limited to, about 2 to 70 wt%.

The transdermally absorbable preparation according to the present invention may be formulated into a patch, a liquid, an ointment, or the like, and may be formulated into, preferably a patch.

Meanwhile, the present invention provides a transdermally therapeutic system including: a drug-containing matrix layer comprising rotigotine and an ethylene-vinyl acetate adhesive at a weight ratio of 1 : 0.1 to 20; and a substrate for supporting the drug-containing matrix layer.

The transdermal therapeutic system of the present invention is characterized by including a drug-containing matrix layer comprising rotigotine and an ethylene-vinyl acetate adhesive at a weight ratio of 1 : 0.1 to 20; and a substrate for supporting the drug-containing matrix layer, and has an effect of treating diseases, such as Parkinson's disease(PD) and restless legs syndrome(RLS), through the permeation of rotigotine into the skin.

The drug-containing matrix layer of the present invention is characterized by including rotigotine as an active drug and an ethylene-vinyl acetate adhesive, and including the rotigotine and the ethylene-vinyl acetate adhesive at a weight ratio of 1 : 0.1 to 20.

In the transdermal therapeutic system of the present invention, the ratio of rotigotine and the ethylene-vinyl acetate adhesive is described in the method for preparing a transdermally absorbable preparation.

The rotigotine and ethylene-vinyl acetate adhesive of the present invention are as described above.

In cases where the proportion of the adhesive is lower than the above numerical range, the stability of rotigotine deteriorates and thus crystals thereof may be precipitated during the long-term storage, and the adhesive strength deteriorates and thus the drug-containing matrix layer has difficulty attaching to the skin. In cases where the proportion of the adhesive is higher than the above numerical range, the adhesive is attached to the skin too hard, causing the adhesive to be an irritant to the skin when the transdermal therapeutic system is attached to and then detached from the skin, the drug release may be delayed, and the skin permeability may deteriorate.

The transdermal therapeutic system of the present invention is characterized by including a substrate for supporting the drug-containing matrix layer. For the substrate, any substrate that can be used for the known patches or the like may be used, and any substrate which is thin and flexible, has no reactivity with the drug-containing matrix layer, and has no reactivity with the skin, causing no allergic reaction, is preferably used as the substrate of the present invention. For example, a non-woven fabric, polyethylene terephthalate, soft polyvinylchloride, polyurethane, polyester, and a silicone coating film may be used as the substrate of the present invention. Preferably, a silicone coating film or polyethylene terephthalate (PET) may be used.

In the transdermal therapeutic system of the present invention, the content of rotigotine may vary depending on the age, body weight, and gender of a patient, the manner of administration, the health condition, and the degree of severity of a disease, and may be applied once a day or divided into multiple doses such that 0.5 to 100 mg of an active material can be administered.

### Advantageous Effects

As set forth above, the present invention provides a method for preparing a transdermally absorbable preparation, the method including a step of mixing rotigotine and an ethylene-vinyl acetate (EVA) adhesive at a weight ratio of 1 : 0.1 to 20, a transdermally absorbable preparation prepared by the method, and a transdermal therapeutic system. The preparation and system of the present invention prevent the precipitation of rotigotine and thus can increase the long-term storage stability, and effectively release rotigotine and thus can be effectively applied to the manufacture of rotigotine-containing patches or the like.

### Brief Description of the Drawings

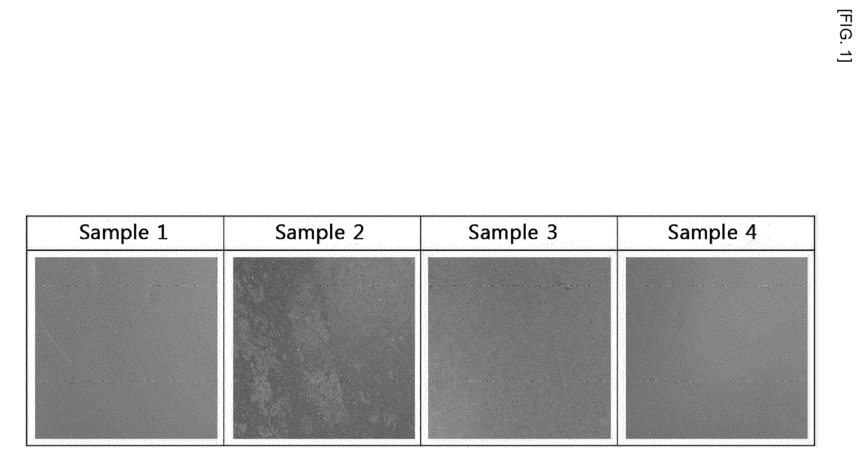

FIG. 1 illustrates images showing observation results of the precipitation of rotigotine crystals according to the kind of adhesive (Sample 1: acrylate adhesive mixed group, Sample 2: silicone adhesive mixed group, Sample 3: polyisobutylene (PIB) adhesive mixed group, and Sample 4: ethylene-vinyl acetate adhesive mixed group).

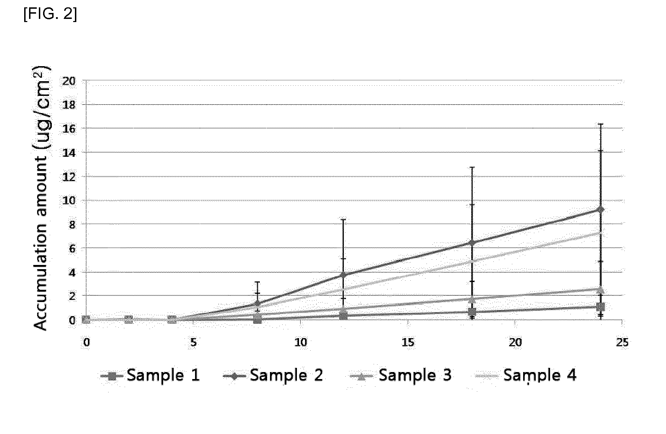

FIG. 2 is a graph showing comparison test results of in vitro skin permeation according to the kind of adhesive (Sample 1: acrylate adhesive mixed group, Sample 2: silicone adhesive mixed group, Sample 3: polyisobutylene (PIB) adhesive mixed group, and Sample 4: ethylene-vinyl acetate adhesive mixed group, X-axis: accumulation amount of rotigotine permeated, Y-axis: time (H)).

### Mode for Carrying Out the Invention

Hereinafter, the present invention will be described in detail with reference to the following examples.

However, the following examples are merely for illustrating the present invention, and are not intended to limit the scope of the present invention.

### <Example 1 >

### Adhesive screening

The miscibility of an adhesive and rotigotine was evaluated following table 1 below. 100 mg of rotigotine and 100 mg of ethanol were respectively put and dissolved, and then each of five kinds of adhesives was weighed to 1.4 g, and then mixed with them at 600 rpm for one hour. The mixture was allowed to stand for one day after the mixing, and then whether or not the rotigotine solution dissolved in the adhesive and ethanol was homogenously mixed was evaluated by the naked eye. For the evaluation, an "X" was marked when the precipitation or layer separation occurred, and an "○" was marked when neither precipitation nor layer separation occurred.

**[Table 1]**

| Classification | Kind of adhesive | Observation result |
|---|---|---|
| Example 1-1 | Acrylate copolymer adhesive(without functional group) | ○ |
| Example 1-2 | Acrylate copolymer adhesive(with -OH group) | ○ |
| Example 1-3 | Acrylate copolymer adhesive(with -COOH group) | ○ |
| Example 1-4 | Poly isobutylene adhesive | ○ |
| Example 1-5 | Acrylic water base adhesive | X |
| Example 1-6 | Silicone adhesive | ○ |
| Example 1-7 | Ethylene vinyl acetate adhesive | ○ |

From test results, as shown in table 1 above, it can be confirmed that, in cases where rotigotine was used together with any other kind of adhesive except the acrylic water base adhesive in example 1-5, homogenous mixtures were obtained.

### <Example 2>

### Long-term stability test according to ingredient - manufacture of patch and evaluation on crystal precipitation

0.3 g of rotigotine and 0.6 g of ethanol were respectively weighed, placed in a 20 mL-volume vial, and then mixed such that rotigotine was completely dissolved. During this procedure, the mixture was slowly stirred under conditions of 60°C, so that the rotigotine can be dissolved. After 1 g of each of the adhesives of table 2 below was placed in the vial containing rotigotine and ethanol, a transdermal administration system was manufactured by the method for preparing a normal transdermally absorbable preparation. Each of the manufactured transdermal administration systems was molded into a circular shape of 10 cm², which was then put in a Petri dish, and then stored in accelerated test conditions of a temperature of 40°C and a relative humidity of 60%. The crystallization of each samples is observed by naked eyes and photographed by conventional digital camera. For the evaluation, an "○" was marked when crystals were precipitated, and an "X" was marked when crystals were not precipitated.

**[Table 2]**

| Test results of crystal precipitation according to the kind of adhesive | | | |
|---|---|---|---|
| Classification | Adhesive | Observation of crystal precipitation after 2 weeks | Observation of crystal precipitation after 4 weeks |
| Sample 1 | Acrylate adhesive | X | X |
| Sample 2 | Silicone adhesive | ○ | ○ |
| Sample 3 | PIB adhesive | ○ | ○ |
| Sample 4 | EVA adhesive | X | X |

As shown in test results table 2 and figure 1, it was confirmed that the storage stability was increased without the precipitation of crystals even under accelerated test conditions when the acryl based adhesive (sample 1) and the EVA adhesive (sample 4) were used rather than when the silicone based adhesive (sample 2) and the PIB adhesive (sample 3) were used.

### <Example 3>

### In vitro skin permeation test

In vitro skin permeation test was conducted on the transdermally absorbable preparations prepared in example 2 using Franz cells (vertical diffusion cells, Hanson research). Each of the prepared transdermally absorbable preparations were molded into a size of 1 cm², which was then fixed on human cadaver skin, and then the test liquid inside the Franz cells, which passed through the skin from the transdermally absorbable preparation and diffused in the skin, was automatically collected every hour. The collected test liquid was pre-treated through centrifugation, and then was analyzed through high-performance chromatography.

The results are shown in FIG. 2, and confirmed the skin permeation of rotigotine according to the kind of adhesive. The silicone based adhesive and the EVA adhesive of samples 2 and 4 showed relatively prompt rotigotine release patterns, and thus it was confirmed that they can be useful as a transdermally absorbable preparation which acts for a relatively short time (within 72 hours).

### <Example 4>

### Manufacture and evaluation of patch according to EVA proportion

The crystallization of rotigotine according to the EVA proportion was evaluated.

Patches were manufactured according to the ingredient contents listed on table 3. More specifically, each of transdermal administration systems was manufactured by the same method as in example 2 according to the normal method for preparing a transdermally absorbable preparation. The manufactured transdermal administration system was molded into a circular shape of 10 cm², which was then put in a Petri dish, and then stored in accelerated test conditions of a temperature of 40°C and a relative humidity of 60%. The precipitation of crystals was observed by the naked eye and photographed by a digital camera. The used adhesive was a styrene based adhesive, and Durotak 87-6911 (Henkel) was used.

**[Table 3]**

| Weight(%) | Comparative example 1 | Example 1-1 | Example 1-2 | Example 1-3 | Example 1-4 |
|---|---|---|---|---|---|
| Rotigotine | 18.8 | 17.6 | 33.0 | 8.8 | 4.7 |
| EVA | 0.0 | 5.9 | 56.0 | 88.2 | 93.8 |
| Adhesive | 81.3 | 76.5 | 0.0 | 0.0 | 0.0 |
| Antioxidant | 0.0 | 0.0 | 5.9 | 5.9 | 1.6 |

As a result of verifying the formation or not of crystals, the crystals were formed in only comparative example 1, and the crystals were not observed even after 4 weeks in examples 1-1 and 1-4. The formation or not of crystals are shown in table 4.

**[Table 4]**

| Test result of crystal precipitation according to EVA proportion | | |
|---|---|---|
| Classification | Observation of crystal precipitation after 2 weeks | Observation of crystal precipitation after 4 weeks |
| Comparative example 1 | ○ | ○ |
| Example 1-1 | X | X |
| Example 1-2 | X | X |
| Example 1-3 | X | X |
| Example 1-4 | X | X |

### Industrial Applicability

Accordingly, the present invention provides a method for preparing a transdermally absorbable preparation, the method including a step of mixing rotigotine and an ethylene-vinyl acetate (EVA) adhesive at a weight ratio of 1 : 0.1 to 20, a transdermally absorbable preparation prepared by the method, and a transdermal therapeutic system. The preparation and system of the present invention prevent the precipitation of rotigotine and thus can increase the long-term storage stability, and effectively release rotigotine and thus can be effectively applied to the manufacture of rotigotine-containing patches or the like. Therefore, the present invention is highly industrially applicable.

## Claims

1. A method for preparing a transdermally absorbable preparation, the method comprising a step of mixing rotigotine and an ethylene-vinyl acetate (EVA) adhesive at a weight ratio of 1 : 0.1 to 20.

2. The method of claim 1, wherein the rotigotine and the ethylene-vinyl acetate adhesive are mixed at a weight ratio of 1 : 0.3 to 20.

3. A transdermally absorbable preparation prepared by the method of claim 1 or 2.

4. A transdermal therapeutic system comprising:
a drug-containing matrix layer comprising rotigotine and an ethylene-vinyl acetate adhesive at a weight ratio of 1 : 0.1 to 20; and
a substrate for supporting the drug-containing matrix layer.

5. The transdermal therapeutic system of claim 4, wherein the drug-containing matrix layer comprises the rotigotine and the ethylene-vinyl acetate adhesive at a weight ratio of 1 : 0.3 to 20.
